(19) Europäisches Patentamt
European Patent Office
Office européen des brevets

(11) Veröffentlichungsnummer: **0 222 292 B1**

# (12) EUROPÄISCHE PATENTSCHRIFT

(45) Veröffentlichungstag der Patentschrift: **21.06.89**

(51) Int. Cl.⁴: **C07C 79/18**, C07C 79/16, C07C 76/02, C07C 91/02, C07C 89/00

(21) Anmeldenummer: **86115203.1**

(22) Anmeldetag: **03.11.86**

## (54) Fluorierte Nitro- und Aminoalkohole.

(30) Priorität: **14.11.85 DE 3540332**

(43) Veröffentlichungstag der Anmeldung: **20.05.87 Patentblatt 87/21**

(45) Bekanntmachung des Hinweises auf die Patenterteilung: **21.06.89 Patentblatt 89/25**

(84) Benannte Vertragsstaaten: **CH DE FR GB LI**

(56) Entgegenhaltungen:
**ANGEWANDTE CHEMIE, Band 98, Nr. 1, Januar 1986, Seiten 96-97, VCH Verlagsgesellschaft mbH, Weinheim, DE; D. SEEBACH et al.: "Di- und trifluorsubstituierte Dilithium-Verbindungen für die organische Synthese"**
**JOURNAL OF PHARMACEUTICAL SCIENCES, Band 56, Nr. 8, August 1967, Seiten 970-973, American Pharmaceutical Association, Washington, DC, US; R.M. PINDER et al.: "Trifluoromethyl analogs of amphetamine and norephedrine"**

(73) Patentinhaber: **BAYER AG, D-5090 Leverkusen 1 Bayerwerk(DE)**

(72) Erfinder: **Beck, Albert K., Loorenstrasse 45, CH-8053 Zürich(CH)**
Erfinder: **Seebach, Dieter, Prof. Dr., Orellistrasse 3, CH-8044 Zürich(CH)**

EP 0 222 292 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft fluorierte Nitroalkohole, fluorierte Aminoalkohole und Verfahren zur Herstellung von fluorierten Nitro- und Aminoalkoholen.

Aus der Gruppe der $CF_3$-substituierten Aminoalkohole ist als einzige Verbindung 2-Amino-3-hydroxy-3-phenyl-1,1,1-trifluorpropan bekannt, das in einer 4-stufigen Synthese mit schlechten Ausbeuten hergestellt werden kann. Diese Verbindung hat eine sehr schwache blutdruckerhöhende Wirkung (siehe J. Pharm. Sciences 56, 971 (1967)).

Es wurden nun fluorierte Nitroalkohole der Formel (I) gefunden

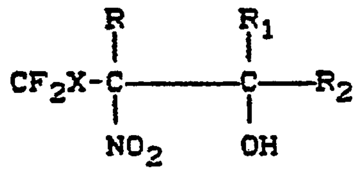

(I),

in der
X für Fluor oder Wasserstoff steht,
R für Wasserstoff oder $C_1$- bis $C_6$-Alkyl steht,
$R_1$ und $R_2$ unabhängig voneinander jeweils für Wasserstoff, gegebenenfalls substituiertes $C_1$- bis $C_6$-Alkyl, gegebenenfalls substituiertes $C_6$- bis $C_{10}$-Aryl oder gegebenenfalls substituiertes, 5 bis 7 Ringatome enthaltendes Heteroaryl stehen, wobei
$R_1$ und $R_2$ auch gemeinsam mit dem C-Atom, an das sie gebunden sind, für einen gegebenenfalls substituierten, gesättigten $C_5$- bis $C_7$-Ring stehen,
in den Fällen, in denen $R_1$ und $R_2$ identisch oder Teil eines unsubstituierten oder symmetrisch substituierten gesättigten $C_5$- bis $C_7$-Rings sind, in der R- und S-Form und beliebigen Mischungen dieser Formen und in den Fällen, in denen $R_1$ und $R_2$ verschieden oder Teil eines unsymmetrisch substituierten gesättigten $C_5$- bis $C_7$-Rings sind, in der R,R-, R,S-, S,R- und S,S-Form und beliebigen Mischungen von zwei, drei und allen dieser Formen.

Als Substituenten an $C_1$- bis $C_6$-Alkyl, $C_6$- bis $C_{10}$-Aryl, 5 bis 7 Ringatome enthaltendem Heteroaryl und gesättigten $C_5$- bis $c_7$-Ringen kommen beispielsweise Nitro-, Halogen-, $C_1$- bis $C_4$-Alkoxy- und CN-Gruppen in Frage. Bevorzugte Halogene sind dabei Fluor, Chlor und Brom, besonders bevorzugte Halogene sind Fluor und Chlor. Bevorzugte $C_1$- bis $C_4$-Alkoxygruppen sind dabei Methoxy-, Ethoxy- und Propoxygruppen, $C_1$- bis $C_6$-Alkyl und gesättigte $C_5$- bis $C_7$-Ringe können auch durch gegebenenfalls substituierte aromatische Gruppen substituiert sein. Dabei sind Phenyl-, p-Nitrophenyl- und 3,4-Methylendioxyphenylgruppen bevorzugt. $C_6$- bis $C_{10}$-Aryl und 5 bis 7 Ringatome enthaltendes Heteroaryl können auch durch $C_1$- bis $C_6$-Alkylgruppen substituiert sein. Dabei sind $C_1$- bis $C_4$-Alkylgruppen, wie Methyl, Ethyl, Propyl, n-Butyl, iso-Butyl und tert.-Butyl, bevorzugt.

In 5 bis 7 Ringatome enthaltendem Heteroaryl können beispielsweise N- und/oder O-Atome vorhanden sein. Vorzugsweise ist ein N-Atom oder ein O-Atom vorhanden, wie im Pyridin- oder Furanringsystem.

In Formel (I) steht X vorzugsweise für Fluor.

In Formel (I) steht R besonders bevorzugt für Wasserstoff oder Methyl, $R_1$ besonders bevorzugt für Wasserstoff und $R_2$ besonders bevorzugt für $C_3$- bis $C_6$-Alkyl oder Phenyl, die gegebenenfalls einen der zuvor beschriebenen Substituenten enthalten. Es ist auch besonders bevorzugt, daß $R_1$ und $R_2$ gemeinsam mit dem C-Atom, an das sie gebunden sind, einen unsubstituierten gesättigten $C_6$-Ring bilden.

Ganz besonders bevorzugte Einzelverbindungen entsprechend der Formel (1) sind in Tabelle 1 aufgeführt.

Tabelle 1

| X | R | $R_1$ | $R_2$ |
|---|---|---|---|
| F | H | H | Phenyl |
| F | H | H | n-Propyl |
| F | H | H | tert.-Butyl |
| F | H | H | n-Hexyl |
| F | H | H | p-Nitrophenyl |
| F | H | H | 3,4-Methylendioxyphenyl |
| F | H | H | $-CH_2-CH_2-$Phenyl |
| F | H | H | $-CH-CH_3$<br>\|<br>Phenyl |
| F | H | $-(CH_2)_5-$ | |
| F | $CH_3$ | H | n-Propyl |
| F | $CH_3$ | H | n-Hexyl |
| F | $CH_3$ | H | $-CH_2-CH_2-$Phenyl |
| H | H | H | Phenyl |
| H | H | H | tert.-Butyl |
| H | H | $-(CH_2)_5-$ | |

Die Verbindungen der Formel (I) weisen in den Fällen, in denen $R_1$ und $R_2$ identisch oder Teil eines unsubstituierten oder symmetrisch substituierten gesättigten $C_5$- bis $C_7$-Rings sind, ein asymmetrisches C-Atom auf. Solche Verbindungen können deshalb in der R-Form, der S-Form und in beliebigen Mischungen dieser Formen vorkommen. In den Fällen, in denen $R_1$ und $R_2$ verschieden oder Teil eines unsymmetrisch substituierten, gesättigten $C_5$- bis $C_7$-Ringes sind, weisen die Verbindungen der Formel (I) zwei asymmetrische C-Atome auf. Solche Verbindungen können deshalb in der R,R-, R,S-, S,R- und S,S-Form und in beliebigen Mischungen von zwei, drei und allen dieser Formen vorkommen.

Die vorliegende Erfindung betrifft die Verbindungen der Formel (I) jeweils sowohl in den einzelnen Formen (entweder R- oder S-Form oder R,R-, R,S-, S,R- oder S,S-Form), als auch in beliebigen Mischungen entweder der R- und S-Form oder der R,R-, R,S-, S,R- und/oder S,S-Form.

Mischungen, welche die R- und S-Form einer Verbindung der Formel (I) mit einem asymmetrischen C-Atom enthalten, können beispielsweise 30 bis 70 Gew.-% der R-Form und ergänzend zu 100 Gew.-% die S-Form enthalten. Mischungen, welche die R,R-, R,S-, S,R- und/oder S,S-Form einer Verbindung der Formel (I) mit zwei asymmetrischen C-Atomen enthalten, können zwei, drei oder alle vier dieser Formen enthalten. In solchen Mischungen kann beispielsweise eine beliebige erste Komponente (R,R-, R,S-, S,R- oder S,S-Form) in einem Anteil von 1 bis 70 Gew.-% und ergänzend zu 100 % eine zweite oder eine zweite und dritte oder eine zweite, dritte und vierte Komponente vorliegen. Vorzugsweise enthalten solche Mischungen alle vier Komponenten, d.h. die R,R-, R,S-, S,R- und S,S-Form einer Verbindung der Formel (I) mit zwei asymmetrischen C-Atomen, wobei jede dieser Komponenten vorzugsweise in einer Menge zwischen 15 und 35 Gew.-% vorliegt und die Summe aller Komponenten 100 Gew.-% gergibt.

Als Beispiele für optisch aktive Formen von Verbindungen der Formel (I) und sie enthaltende Mischungen seien genannt; R,R-1,1,1-Trifluor-2-nitro-3-phenyl-propan-3-ol, R,S-1,1,1-Trifluor-2-nitro-3-phenyl-propan-3-ol, S,R-1,1,1-Trifluor-2-nitro-3-phenyl-propan-3-ol, S,S,-1,1,1-Trifluor-2-nitro-3-phenyl-propan-3-ol und Gemische aller vier dieser Einzelformen, wobei jede Einzelform einen Anteil am Gesamtgemisch zwischen 15 und 35 Gew.-% hat und die Summe aller vier Komponenten 100 Gew.-% ergibt, sowie R-1-Hydroxy-1-(2,2,2-trifluor-1-nitro-ethyl)-cyclohexan, S-1-Hydroxy-1-(2,2,2-trifluor-1-nitro-ethyl)-cyclohexan und Gemische dieser Cyclohexane, die 30 bis 70 Gew.-% der R-Komponente und ergänzend zu 100 Gew.-% die S-Komponente enthalten.

Die Zusammensetzung eines vorliegenden Gemisches optischer Isomerer einer Verbindung der Formel (I) kann auf an sich bekannte Weise verändert werden. Beispielsweise kann man, sofern R für Wasserstoff steht, die OH-Gruppe am benachbarten C-Atom schützen, z.B. durch Überführung in den entsprechenden Trimethylsilylether, anschließend eine Deprotonierung durchführen, gefolgt von einer Protonierung, vorzugsweise bei Temperaturen im Bereich -90 bis -100°C mit Eisessig, und schließlich die Schutzgruppe wieder abspalten. Wenn R für $C_1$-bis $C_6$-Alkyl steht kann man die Zusammensetzung eines vorliegenden Gemisches optischer Isomerer einer Verbindung der Formel (I) beispielsweise dadurch ändern, daß man eine Oxidation durchführt und anschließend reduziert. Unabhängig von den Resten R, $R_1$ und $R_2$ kann dies auch mit chromatographische Methoden erreicht werden. Reine optische Isomere von Verbindungen der Formel (I) können aus Gemischen optischer Isomerer ebenfalls auf an sich bekannte Weise erhalten werden, z.B. durch Hochdruckflüssigkeitschromatographie (HPLC).

Es wurde weiterhin ein Verfahren zur Herstellung von fluorierten Nitroalkoholen der Formel (I)

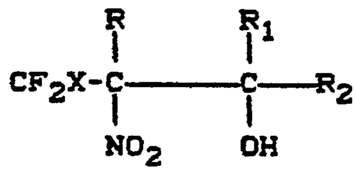 (I),

in der
X für Fluor steht,
R für Wasserstoff oder $C_1$- bis $C_6$-Alkyl steht,
$R_1$ und $R_2$ unabhängig voneinander jeweils für Wasserstoff, gegebenenfalls substituiertes $C_1$- bis $C_6$-Alkyl, gegebenenfalls substituiertes $C_6$- bis $C_{10}$-Aryl oder gegebenenfalls substituiertes, 5 bis 7 Ringatome enthaltendes Heteroaryl stehen, wobei
$R_1$ und $R_2$ auch gemeinsam mit dem C-Atom, an das sie gebunden sind, für einen gegebenenfalls substituierten, gesättigten $C_5$- bis $C_7$-Ring stehen,
gefunden, das dadurch gekennzeichnet ist, daß man Fluornitroalkane der Formel (II)

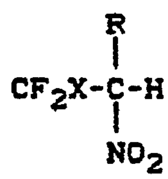 (II),

in der
X für Fluor steht und
R die bei Formel (I) angegebene Bedeutung hat,
mit Carbonylverbindungen der Formel (III)

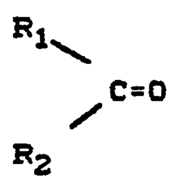 (III),

in der
$R_1$ und $R_2$ die bei Formel (I) angegebene Bedeutung haben,
in Gegenwart von Kaliumfluorid oder Aluminiumoxid umsetzt.

In dieses erfindungsgemäße Verfahren zur Herstellung von fluorierten Nitroalkoholen der Formel (I) können z.B. solche Fluornitroalkane der Formel (II) eingesetzt werden, die gemäß der DE-OS 3 305 201 oder DE-OS 3 305 202 erhalten worden sind. Bevorzugt ist der Einsatz von Fluornitroalkanen der Formel (II), bei denen R für Wasserstoff oder Methyl steht.

Von den Carbonylverbindungen der Formel (III) sind solche bevorzugt, bei denen $R_1$ und $R_2$ die Bedeutungen haben, die bei der Erläuterung der neuen fluorierten Nitroalkohole der Formel (I) als bevorzugt für $R_1$ und $R_2$ angegeben sind. Besonders bevorzugte Carbonylverbindungen der Formel (III) sind Benzaldehyd, n-Butyraldehyd, 2,2,2-Trimethylacetaldehyd, n-Heptanal, p-Nitrobenzaldehyd, 3,4-Me-

thylendioxybenzaldehyd, 3-Phenylpropanal, 2-Phenylpropanal und Cyclohexanon.

Die Carbonylverbindungen der Formel (III) sind gut zugängliche, zum größten Teil im Handel erhältliche Produkte.

Obwohl eine Verbindung der Formel (II) mit einer Verbindung der Formel (III) im molaren Verhältnis von 1:1 reagiert, ist es bevorzugt, eine dieser beiden Reaktionskomponenten im Überschuß einzusetzen, beispielsweise im Molverhältnis von 2:1 bis 1:2. Da häufig die Carbonylverbindung der Formel (III) besser zugänglich ist, ist es für Umsetzungen im technischen Maßstab im allgemeinen vorteilhaft, die Verbindung der Formel (III) im Überschuß einzusetzen, beispielsweise in einem molaren Verhältnis zur Verbindung der Formel (II) von 1,2 bis 2:1.

Dieses erfindungsgemäße Verfahren wird in Gegenwart von Kaliumfluorid oder Aluminiumoxid durchgeführt. Das Kaliumfluorid kann dabei in technischer oder chemisch reiner Form und wasserfrei oder wasserhaltig eingesetzt werden. Vorzugsweise wird das Kaliumfluorid in chemisch reiner, wasserfreier Form verwendet. Das Aluminiumoxid kann dabei sauer, neutral oder basisch und von beliebiger Aktivität sein. Vorzugsweise wird handelsübliches basisches Aluminiumoxid der Aktivitätsstufe I in Form feiner Pulver verwendet. Kaliumfluorid kann beispielsweise in Mengen von 0,05 bis 100 Mol-%, vorzugsweise 0,1 bis 70 Mol-%, bezogen auf die Verbindung der Formel (II), eingesetzt werden. Aluminiumoxid kann beispielsweise in Mengen von 0,1 bis 500 Mol-%, vorzugsweise 10 bis 200 Mol-%, bezogen auf die Verbindung der Formel (II), eingesetzt werden.

Für dieses erfindungsgemäße Verfahren geeignete Temperaturen sind beispielsweise solche zwischen 0°C und dem Siedepunkt (bei Normaldruck) der am niedrigsten siedenden Komponente des Reaktionsgemisches. Besonders bevorzugt und einfach ist es, dieses erfindungsgemäße Verfahren bei Raumtemperatur, d.h. bei 20 bis 25°C Umgebungstemperatur durchzuführen.

Im allgemeinen wird dieses erfindungsgemäße Verfahren bei Normaldruck durchgeführt. Man kann jedoch auch erniedrigte, erhöhte Drucke und Hochdrucke anwenden, beispielsweise solche im Bereich von 0,5 bar bis 15 kbar.

Dieses erfindungsgemäße Verfahren kann in Anwesenheit oder in Abwesenheit von Lösungsmitteln durchgeführt werden. Falls Lösungsmittel eingesetzt werden sollen, können hierfür beispielsweise Alkohole verwendet werden, insbesondere Isopropanol. Der Einsatz von Alkoholen als Lösungsmittel ist im allgemeinen dann vorteilhaft, wenn ein Fluonitroalkan der Formel (II) mit R = $C_1$- bis $C_6$-Alkyl und/oder eine Carbonylverbindung der Formel (III) mit $R_1$ und/oder $R_2$= gegebenenfalls substituiertes $C_6$- bis $C_{10}$-Aryl oder gegebenenfalls substituiertes 5 bis 7 Ringatome enthaltendes Heteroaryl eingesetzt und in Gegenwart von Kaliumfluorid gearbeitet wird.

Während dieser erfindungsgemäßen Umsetzung wird vorteilhafterweise gerührt. Man kann die Umsetzung jedoch auch häufig einfach durch Stehenlassen des jeweiligen Ansatzes zu Ende bringen. Die Reaktionszeit kann beispielsweise im Bereich von 30 Minuten bis 30 Stunden liegen, vorzugsweise im Bereich von 20 bis 25 Stunden.

Das nach dieser erfindungsgemäßen Umsetzung vorliegende Gemisch kann beispielsweise wie folgt aufgearbeitet werden:

Zunächst werden feste Bestandteile des Reaktionsgemisches abgetrennt, z.B. durch Filtration, danach gegebenenfalls vorhandene leichter flüchtige Bestandteile de Reaktionsgemisches, z.B. Lösungsmittel und/oder nicht umgesetztes fluoriertes Nitroalken der Formel (II), was z.B. durch Abdampfen, gegebenenfalls bei vermindertem Druck, erfolgen kann. Danach wird mit einem mit Wasser nicht mischbaren Lösungsmittel, z.B. Diethylether, und Wasser versetzt, wobei gegebenenfalls vorhandene nicht umgesetzte Carbonylverbindung der Formel (III) durch den Einsatz einer wäßrigen $NaHSO_3$-Lösung anstelle von Wasser beseitigt werden kann. Danach wird erneut filtriert, die Lösungsmittelphase von der Wasserphase getrennt, aus der Lösungsmittelphase das Lösungsmittel entfernt, z.B. durch Abdampfen, und so die Verbindung der Formel (I) erhalten. Für viele Verwendungen ist die so isolierte Verbindung der Formel (I) rein genug. Falls gewünscht, kann sie z.B. durch fraktionierte Destillation, gegebenenfalls bei vermindertem Druck, weiter gereinigt werden.

Im allgemeinen fallen die fluorierten Nitroalkohole der Formel (I) als Gemisch der diastereomeren Enantiomerenpaare an. Die Isomerenverteilung in einer solchen Mischung kann verändert und die Abtrennung einzelner Isomerer aus einer solchen Mischung vorgenommen werden, wie weiter oben beschrieben.

Es wurde noch ein weiteres Verfahren zur Herstellung von fluorierten Nitroalkoholen der Formel (I)

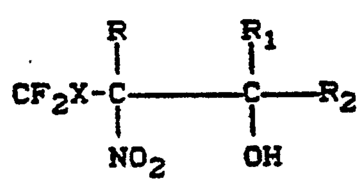

(I),

in der

X für Fluor oder Wasserstoff steht,

R für Wasserstoff steht und
$R_1$ und $R_2$ die weiter oben angegebene Bedeutung haben,
gefunden, das dadurch gekennzeichnet ist, daß man zunächst Fluornitroalkane der Formel (II)

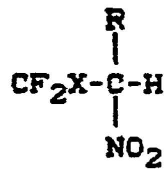 (II),

in der
X für Fluor oder Wasserstoff und
R für Wasserstoff steht
in Gegenwart eines aprotischen Lösungsmittels und gegebenenfalls eines Kosolvens mit einer Lithium-Alkyl-Verbindung bei Temperaturen im Bereich -80 bis -130°C umsetzt, dem Reaktionsgemisch anschließend bei -80 bis -130°C eine Carbonylverbindung der Formel (III)

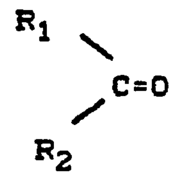 (III),

in der
$R_1$ und $R_2$ die weiter oben angegebene Bedeutung haben,
zusetzt, anschließend auf -60 bis -80°C erwärmt, dann bei -80 bis -130°C mit Eisessig und einem aprotischen Lösungsmittel versetzt und abschließend auf eine Temperatur über -20°C erwärmt.

Die bevorzugten und besonders bevorzugten Bedeutungen von $R_1$ und $R_2$ in den Formeln (I), (II) und (III) sind wie weiter oben angegeben.

Als aprotische Lösungsmittel für dieses erfindungsgemäße Verfahren kommen beispielsweise cyclische Ether in Frage. Bevorzugt ist Tetrahydrofuran. Vorzugsweise wird die Umsetzung mit einer Lithium-Alkyl-Verbindung in Gegenwart eines Kosolvens durchgeführt. Hierfür geeignet sind beispielsweise cyclische Harnstoffderivate, wie N,N-Dimethylpropylenharnstoff und N,N-Dimethylethylenharnstoff, sowie Hexamethylphosphorsäuretriamid. Bevorzugt ist N,N-Dimethylpropylenharnstoff.

Geeignete Lithium-Alkyl-Verbindungen sind beispielsweise solche, in denen der Alkylrest 1 bis 4 C-Atome aufweist und geradkettig ist. Bevorzugt ist n-Butyl-lithium.

Innerhalb des Temperaturbereichs von -80 bis -130°C ist der Bereich von -90 bis -100°C bevorzugt, innerhalb des Temperaturbereiches von -60 bis -80°C derjenige von -65 bis -75°C. Die abschließende Erwärmung auf eine Temperatur über -20°C kann beispielsweise auf eine Temperatur bis zu +50°C erfolgen. Bevorzugte Endtemperaturen sind solche zwischen +5 und +20°C.

Auf 1 Mol eines fluorierten Nitroalkans der Formel (II) können hier beispielsweise 500 bis 5000 ml eines aprotischen Lösungsmittels, 500 bis 2000 ml eines Kosolvens, 1,5 bis 2,5 Mole Lithium-Alkyl-Verbindung, 0,8 bis 1,5 Mole Carbonylverbindung der Formel (III) und 1,5 bis 2,5 Mole Eisessig eingesetzt werden.

Die Aufarbeitung des nach der Erwärmung auf eine Temperatur über -20°C vorliegenden Gemisches kann beispielsweise so erfolgen, daß man dieses in ein Diethylether/Wasser-Gemisch schüttet, die Etherphase abtrennt, mit viel Wasser wäscht (zur Entfernung des gegebenenfalls vorhandenen Kosolvens), danach die Etherphase eindampft und so den fluorierten Nitroalkohol der Formel (I) erhält.

Weiterhin wurden neue fluorierte Aminoalkohole der Formel (IV) gefunden

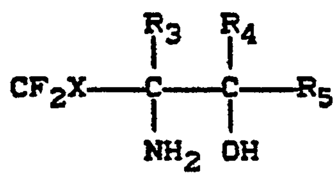 (IV)

in der
X für Fluor oder Wasserstoff steht,
$R_3$ für Wasserstoff oder $C_1$- bis $C_6$-Alkyl steht,

$R_4$ und $R_5$ unabhängig voneinander jeweils für Wasserstoff, gegebenenfalls substituiertes $C_1$- bis $C_6$-Alkyl, gegebenenfalls substituiertes $C_6$- bis $C_{10}$-Aryl oder gegebenenfalls substituiertes 5 bis 7 Ringatome enthaltendes Heteroaryl stehen,
wobei die
Kombination X = F/$R_3$ =H/$R_4$ = H/$R_5$ = unsubstituiertes Phenyl ausgenommen ist.
und wobei $R_4$ und $R_5$ auch gemeinsam mit dem C-Atom, an das sie gebunden sind, für einen gegebenenfalls substituierten, gesättigten $C_5$- bis $C_7$-Ring stehen,
in den Fällen, in denen $R_4$ und $R_5$ identisch oder Teil eines unsubstituierten oder symmetrisch substituierten gesättigten $C_5$- bis $C_7$-Ringes sind, in der R- und S-Form und beliebigen Mischungen dieser Formen und in Fällen, in denen $R_4$ und $R_5$ verschieden oder Teil eines unsymmetrisch substituierten $C_5$- bis $C_7$-Ringes sind, in der R,R-, R,S-, S,R- und S,S,-Form und beliebigen Mischungen von zwei, drei und allen vier dieser Formen.

Als Substituenten an $C_1$- bis $C_6$-Alkyl, $C_6$- bis $C_{10}$-Aryl, 5 bis 7 Ringatome enthaltendem Heteroaryl und gesättigten $C_5$- bis $C_7$-Ringen kommen beispielsweise Nitro, Halogen-, $C_1$- bis $C_4$-Alkoxy- und CN-Gruppen in Frage. Bevorzugte Halogene sind dabei Fluor, Chlor und Brom, besonders bevorzugte Halogene sind Fluor und Chlor. Bevorzugte $C_1$- bis $C_4$-Alkoxygruppen sind dabei Methoxy-, Ethoxy- und Propoxygruppen. $C_1$- bis $C_6$-Alkyl und gesättigte $C_5$- bis $C_7$-Ringe können auch durch gegebenenfalls substituierte aromatische Gruppen substituiert sein. Dabei sind Phenyl-, p-Nitrophenyl- und 3,4-Methylendioxyphenylgruppen bevorzugt. $C_6$- bis $C_{10}$-Aryl und 5 bis 7 Ringatome enthaltendes Heteroaryl können auch durch $C_1$- bis $C_6$-Alkylgruppen substituiert sein. Dabei sind $C_1$- bis $C_4$-Alkylgruppen, wie Methyl, Ethyl, Propyl, n-Butyl, iso-Butyl und tert.-Butyl, bevorzugt.

In 5 bis 7 Ringatome enthaltendem Heteroaryl können beispielsweise N- und/oder O-Atome vorhanden sein. Vorzugsweise ein N/Atom oder ein O-Atom vorhanden, wie im Pyridin- oder Furanringsystem.

In Formel (IV) steht $R_3$ besonders bevorzugt für Wasserstoff oder Methyl, $R_4$ besonders bevorzugt für Wasserstoff und $R_5$ besonders bevorzugt für $C_3$- bis $C_6$-Alkyl und, falls $R_3$ und/oder $R_4$ nicht Wasserstoff ist, auch für Phenyl. Ganz besonders bevorzugte Einzelverbindungen entsprechen der Formel (IV) mit X = Fluor und sind in Tabelle 2 angeführt.

**Tabelle 2**

| $R_3$ | $R_4$ | $R_5$ |
|---|---|---|
| H | H | n-Propyl |
| H | H | tert.-Butyl |
| H | H | n-Hexyl |
| H | H | p-Nitrophenyl |
| H | H | 3,4-Methylendioxyphenyl |
| H | H | $-CH_2-CH_2-$Phenyl |
| H | H | $-CH(CH_3)-$Phenyl |
| H | $-(CH_2)_5-$ | |
| $CH_3$ | H | n-Propyl |
| $CH_3$ | H | n-Hexyl |
| $CH_3$ | H | $-CH_2-CH_2-$Phenyl |

Auch die Verbindungen der Formel (IV) weisen in den Fällen, in denen $R_4$ und $R_5$ identisch oder Teil eines unsubstituierten oder symmetrisch substituierten gesättigten $C_5$- bis $C_7$-Ringes sind ein asymmetrisches C-Atom auf. Solche Verbindungen können deshalb in der R-Form, der S-Form und in beliebigen Mischungen dieser Formen vorkommen. In den Fällen, in denen $R_4$ und $R_5$ verschieden oder Teil eines

unsymmetrisch substituierten gesättigten $C_5$- bis $C_7$-Ringes sind weisen die Verbindungen der Formel (IV) zwei asymmetrische C-Atome auf. Solche Verbindungen können deshalb in der R,R-, R,S-, S,R- und S,S-Form und in beliebigen Mischungen von zwei, drei und allen dieser Formen vorkommen.

Die vorliegende Erfindung betrifft die Verbindungen der Formel (IV) jeweils sowohl in den einzelnen Formen (entweder R- oder S-Form oder R,R-, R,S-, S,R- oder S,S-Form), als auch in beliebigen Mischungen entweder der R- und S-Form oder der R,R-, R,S-, S,R- und/oder S,S-Form.

Mischungen, welche die R- und S-Form einer Verbindung der Formel (IV) mit einem asymmetrischen C-Atom enthalten, können biespielsweise 30 bis 70 Gew.-% der R-Form und ergänzend zu 100 Gew.-% die S-Form enthalten. Mischungen, welche die R,R-, R,S-, S,R- und/oder S,S-Form einer Verbindung der Formel (IV) mit zwei asymmetrischen C-Atomen enthalten, können zwei, drei oder alle vier dieser Formen enthalten. In solchen Mischungen kann beispielsweise eine beliebige erste Komponente (R,R-, R,S-, S,R- oder S,S-Form) in einem Anteil von 1 bis 70 Gew.-% und ergänzend zu 100 % eine zweite oder eine zweite und dritte oder eine zweite, dritte und vierte Komponente vorliegen. Vorzugsweise enthalten solche Mischungen alle vier Komponenten, d.h. die R,R-, R,S-, S,R- und S,S-Form einer Verbindung der Formel (IV) mit zwei asymmetrischen C-Atomen, wobei jede dieser Komponenten vorzugsweise in einer Menge zwischen 15 und 35 Gew.-% vorliegt und die Summe aller Komponenten 100 Gew.-% ergibt.

Als Beispiele für optisch aktive Formen von Verbindungen der Formel (IV) und sie enthaltende Mischungen seien genannt: R,R-1,1,1-Trifluor-2-methyl-2-amino-3-phenyl-propan-3-ol, R,S-1,1,1-Trifluor-2-methyl-2-amino-3-phenyl-propan-3-ol, S,R-1,1,1-Trifluor-2-methyl-2-amino-3-phenyl-propan-3-ol, S,S-1,1,1-Trifluor-2-methyl-2-amino-3-phenyl-propan-3-ol und Gemische aller vier dieser Einzelformen, wobei jede Einzelform einen Anteil am Gesamtgemisch zwischen 15 und 35 Gew.-% hat und die Summe aller vier Komponenten 100 Gew.-% ergibt, sowie R-1-Hydroxy-1-(2,2,2-trifluor-1-amino-ethyl)-cyclohexan, S-1-Hydroxy-1-(2,2,2-trifluor-1-amino-ethyl)-cyclohexan und Gemische dieser Cyclohexane, die 30 bis 70 Gew.-% der R-Komponente und ergänzend zu 100 Gew.-% die S-Komponente enthalten.

Die Zusammensetzung eines vorliegenden Gemisches optischer Isomerer einer Verbindung der Formel (IV) kann auf an sich bekannte Weise verändert und reine optische Isomere von Verbindungen der Formel (IV) können aus Gemischen optischer Isomerer ebenfalls auf an sich bekannte Weise erhalten werden, z.B. durch Hochdruckflüssigkeitschromatographie (HPLC) oder fraktionierte Kristallisation.

Es wurde weiterhin ein Verfahren zur Herstellung von fluorierten Aminoalkoholen der Formel (V)

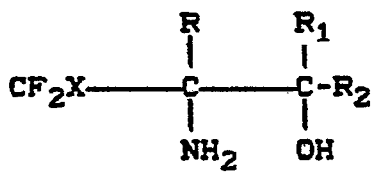

(V)

in der

X für Fluor oder Wasserstoff steht,

R für Wasserstoff oder $C_1$- bis $C_6$-Alkyl steht,

$R_1$ und $R_2$ unabhängig voneinander jeweils für Wasserstoff, gegebenenfalls substituiertes $C_1$- bis $C_6$-Alkyl, gegebenenfalls substituiertes $C_6$- bis $C_{10}$-Aryl oder gegebenenfalls substituiertes, 5 bis 7 Ringatome enthaltendes Heteroaryl stehen, wobei

$R_1$ und $R_2$ auch gemeinsam mit dem C-Atom, an das sie gebunden sind, für einen gegebenenfalls substituierten, gesättigten $C_5$- bis $C_7$-Ring stehen,

gefunden, das dadurch gekennzeichnet ist, daß man fluorierte Nitroalkohole der Formel (I)

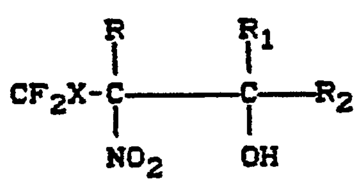

(I)

in der

X für Fluor oder Wasserstoff steht,

R, $R_1$ und $R_2$ die bei Formel (V) angegebene Bedeutung haben,

hydriert.

Die Ausgangsprodukte für dieses Verfahren,die fluorierten Nitroalkohole der Formel (I), können wie weiter oben beschrieben, z.B. durch Umsetzung von fluorierten Nitroalkanen der Formel (II)

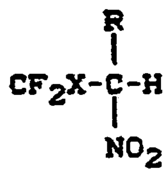 (II),

in der
R die bei Formel (V) angegebene Bedeutung hat,
mit Carbonylverbindungen der Formel (III)

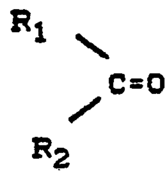 (III)

in der
$R_1$ und $R_2$ die bei Formel (V) angegebene Bedeutung haben,
in Gegenwart von Kaliumfluorid oder Aluminiumoxid erhalten werden.

Die erfindungsgemäße Hydrierung kann beispielsweise katalytisch mit Wasserstoff oder durch Umsetzung mit Metallhydriden durchgeführt werden.

Eine derartige katalytische Hydrierung mit Wasserstoff kann in Gegenwart oder in Abwesenheit von Lösungsmittel durchgeführt werden. Im allgemeinen ist es vorteilhaft, in Gegenwart eines Lösungsmittels zu arbeiten, da dann eine bessere Kontrolle der exotherm verlaufenden Hydrierung möglich ist.

Als Lösungsmittel kommen beispielsweise inerte organische Lösungsmittel in Frage. Geeignet sind beispielsweise Alkohole wie Methanol, Ethanol, Ethylenglykol und Diethylenglykol, Ether wie Dioxan, Tetrahydrofuran, Ethylenglykolmonomethylether, Ethylenglykolmonoethylether, Ethylenglykoldimethylether, Diethylenglykolmonoethylether und Diethylenglykoldimethylether, gesättigte Kohlenwasserstoffe wie Cyclohexan oder Ester wie Essigsäureethylester.

Bevorzugt wird als Lösungsmittel Methanol oder Ethanol eingesetzt. In diesem Fall ist im allgemeinen eine problemlose destillative Abtrennung der Lösungsmittel von den Hydrierprodukten möglich.

Die erfindungsgemäße katalytische Hydrierung mit Wasserstoff kann in Reaktionsapparaten durchgeführt werden, die für Hydrierungen bei Normaldruck und/oder Hydrierungen unter Druck geeignet sind. Geeignete Materialien für die Reaktionsapparaturen sind beispielswiese Glas, Emaille, Stahl oder Edelstahl.

Der Wasserstoffdruck, unter dem die Hydrierung durchgeführt wird, kann in weiten Grenzen variieren. Bevorzugt ist ein Druck von 1 bis 30 bar, besonders bevorzugt von 5 bis 20 bar.

Die Reaktionstemperatur kann ebenfalls in weiten Grenzen variiert werden und beispielsweise zwischen 0 und 120°C liegen. Besonders bevorzugt sind Temperaturen zwischen 10 und 80°C, ganz besonders bevorzugt solche zwischen 20 und 60°C.

Die Menge Wasserstoff kann ebenfalls in weiten Grenzen variiert werden. Vorzugsweise werden pro Mol der Verbindung der Formel (I) mindestens 3 Mole Wasserstoff eingesetzt. Falls prinzipiell weitere Reaktionen mit Wasserstoff möglich sind, z.B. die Hydrierung einer weiteren vorhandenen Nitrogruppe, und solche weiteren Reaktionen mit Wasserstoff unerwünscht sind, ist es vorteilhaft, größere Überschüsse an Wasserstoff zu vermeiden, z.B. mehr als 8 Mole Wasserstoff pro Mol der Verbindung der Formel (I) einzusetzen.

Die für die erfindungsgemäße katalytische Hydrierung mit Wasserstoff erforderliche Reaktionszeit ist abhängig von der Reaktionsgeschwindigkeit, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden.

Geeignete Katalysatoren für die erfindungsgemäße katalytische Hydrierung mit Wasserstoff sind beispielsweise solche, die aus Metallen und/oder Verbindungen von Elementen der 8. Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Oxide, Hydroxide und/oder Oxidhydrate handeln. Zusätzlich können weitere Metalle, z.B. Kupfer, Vanadin, Molybdän, Chrom und/oder Mangan, sowie Verbindungen dieser Metalle zugegen sein.

Solche Hydrierkatalysatoren können ausschließlich oder überwiegend aus dem zuvor beschriebenen Wasserstoff-übertragenden Substanzen bestehen, diese können aber auch auf Trägermaterialien aufgebracht sein. Als Trägermaterialien für die Wasserstoff-übertragenden Substanzen kommen beispielsweise in Frage anorganische Materialien wie Kiesel gur, Kieselsäure, Aluminiumoxide, Alkali- und Erdalkalisilikate, Aluminiumsilikate, Montmorillonit Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest,

Aktivkohle oder Bariumsulfat, aber auch organische Materialien, beispielsweise natürlich vorkommende oder synthetische Verbindungen mit hohem Molekulargewicht, wie Seide, Polyamide, Polystyrole, Zellstoff oder Polyurethane. Bevorzugt sind anorganische Trägermaterialien. Das Trägermaterial kann beispielswiese in Form von Kugeln, Strängen, Fäden, Zylindern, Polygonen oder in Pulverform vorliegen.

Derartige Trägerkatalysatoren können im allgemeinen 0,5 bis 50 Gew.-%, vorzugsweise 1 bis 10 Gew.-% der Wasserstoff-übertragenden Substanz, bezogen auf die Gesamtmasse des Trägerkatalysators, enthalten. Die Wasserstoff-übertragende Substanz kann dabei homogen im Trägermaterial verteilt sein. Bevorzugt sind jedoch Katalysatoren, in deren äußerer Schicht oder auf deren Oberfläche die Wasserstoff-übertragende Substanz abgelagert ist. DieHerstellung und die Formgebung von Katalysatoren die für die erfindungsgemäße katalytische Hydrierung mit Wasserstoff Verwendung finden können, kann in an sich bekannter Weise erfolgen (siehe beispielsweise Houben-Weyl, Methoden der organischen Chemie, Band VI, 1c, Teil I, Seiten 16 bis 26, Georg Thieme Verlag, Stuttgart, 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Kohle, Palladium auf Aluminiumoxid, Palladium auf Calciumcarbonat, Palladium auf Bariumsulfat, Palladium auf Kieselsäure, Platin auf Kohle und Platin auf Aluminiumoxid.

Bevorzugte Hydrierkatalysatoren, die ausschließlich oder überwiegend aus Wasserstoff-übertragender Substanz bestehen, sind beispielsweise oxidische Katalysatoren wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit z.B. Alkalihydriden, Alkaliboranaten, Metallalkylen, Hydrazin, Formaldehyd, Wasserstoff oder elektropositiveren Metallen herstellbare Schwarzkatalysatoren, wie Palladium-Schwarz, Platin-Schwarz und Rhodium-Schwarz.

Besonders bevorzugte Katalysatoren für die erfindungsgemäße katalytische Hydrierung mit Wasserstoff sind Palladium auf Kohle, Palladium auf Aluminiumoxid, Palladium auf Kieselsäure und Palladium auf Calciumcarbonat.

Für die erfindungsgemäße katalytische Hydrierung mit Wasserstoff können auch Skelettkatalysatoren vom Raney-Typ eingesetzt werden, wobei Raney-Nickel bevorzugt ist. In diesem Falle ist jedoch darauf zu achten, daß nur sorgfältig neutral gewaschenes Raney-Nickel zum Einsatz gelangt, da sonst unerwünschte Nebenreaktionen stattfinden können.

Der Katalysator für die erfindungsgemäße katalytische Hydrierung mit Wasserstoff kann beispielsweise in Mengen von 0,1 bis 150 Gew.-% Wasserstoff-übertragende Substanz, bezogen auf die Verbindung der Formel (I) eingesetzt werden. Für Ansätze in technischem Maßstab werden vorzugsweise 0,1 bis 20 Gew.-%, besonders bevorzugt 0,2 bis 5 Gew.-% Wasserstoff-übertragende Substanz, bezogen auf die Verbindung der Formel (I) eingesetzt.

Zur Durchführung der erfindungsgemäßen katalytischen Hydrierung mit Wasserstoff können auch Gemische aus zwei oder mehreren der genannten Hydrierkatalysatoren verwendet werden.

Die katalytische Aktivität der Hydrierkatalysatoren bleibt bei der Durchführung des erfindungsgemäßen Verfahrens im allgemeinen weitgehend erhalten, so daß diese bei diskontinuierlicher Arbeitweise wiederholt eingesetzt werden und bei kontinuierlicher Arbeitsweise längere Zeit in Gebrauch bleiben können.

In einer einfachen diskontinuierlichen Ausführungsform kann die erfindungsgemäße katalytische Hydrierung mit Wasserstoff beispielsweise wie folgt durchgeführt werden: Ein mit einer Rühr- oder Mischeinrichtung versehener, temperierbarer Autoklav wird mit der einzusetzenden Verbindung der Formel (I), dem Hydrierkatalysator und einem Lösungsmittel beschickt. Danach wird Wasserstoff bis zum gewünschten Druck aufgedrückt und das Gemisch unter intensiver Durchmischung auf die gewünschte Reaktionstemperatur erhitzt. Der Reaktionsverlauf läßt sich leicht durch die Messung des Wasserstoffverbrauchs verfolgen. Wenn die theoretische Menge Wasserstoff aufgenommen worden ist, kann die Hydrierung abgebrochen werden, z.B. durch Ab kühlung, Beendigung der Durchmischung, Entspannung und/oder Beseitigung der Wasserstoffatmosphäre. Die Aufarbeitung des Reaktionsgemisches kann beispielsweise so erfolgen, daß man zunächst den Katalysator abfiltriert, danach das Lösungsmittel abdestilliert und die verbleibenden Reaktionsprodukte gegebenenfalls durch Destillation oder Kristallisation reinigt. Die erfindungsgemäße katalytische Hydrierung mit Wasserstoff kann auch kontinuierlich durchgeführt werden.

Die erfindungsgemäße Hydrierung kann aber auch durch Umsetzung mit Metallhydriden durchgeführt werden. Hierfür geeignete Metallhydride sind z.B. Alkali- und/oder Erdalkalihydride, auch solche mit Gehalten an weiteren Metallen. Ein bevorzugtes Metallhydrid ist Lithiumaluminiumhydrid. Vor der Umsetzung der Verbindung der Formel (I) mit Metallhydriden ist es im allgemeinen erforderlich, die OH-Gruppe der Verbindung der Formel (I) zu schützen. Dies kann z.B. in an sich bekannter Weise durch Bildung der entsprechenden Silylether und THP- oder Alkoxyethylderivate geschehen, besonders geeignet sind die Trimethyl- und die t.-Butyl-dimethylsilylether.

Das Metallhydrid wird, bezogen auf die Verbindung der Formel (I),vorzugsweise in stöchiometrischem Überschuß eingesetzt.

Die erfindungsgemäße Hydrierung mit Metallhydriden wird vorzugsweise in einem inerten Lösungsmittel durchgeführt, Geeignet hierfür sind beispielsweise Ether. Diethylether wird hierfür bevorzugt.

Für solche Hydrierungen mit Metallhydriden sind z.B. Temperaturen zwischen 0°C und der Siedetemperatur des Lösungsmittels geignet. Wenn Diethylether als Lösungsmittel verwendet wird, ist es bevorzugt, die Hydrierung bei am Rückfluß siedendem Diethylether durchzuführen.

Die neuen fluorierten Aminoalkohole der Formel (IV) sind Pharmazeutika und Pflanzenschutzmittel, insbesondere Insektizide.

Die neuen fluorierten Aminoalkohole der Formel (IV) und das bekannte 2-Amino-3-hydroxy-3-phenyl-1,1,1-trifluorpropan lassen sich erfindungsgemäß auf einfache Weise und in guten ausbeuten herstellen.

Die neuen fluorierten Nitroalkohole der Formel (I) können, wie beschrieben, als Zwischenprodukte zur Herstellung von fluorierten Aminoalkoholen der Formel (V) verwendet werden.

Beispiel

I. Allgemeine Arbeitsvorschriften

A. Allgemeine Arbeitsvorschrift zur Herstellung von Nitroalkoholen der Formel (I) unter Verwendung von Kaliumfluorid und Isopropanol.

Es wurden in einem 250 ml Rundkolben 184 mMol eines Fluornitroalkans in 100 ml Isopropanol gelöst, 78,6 mMol einer Carbonylverbindung der allgemeinen Formel (III) und 6,8 mMol KF zugegeben und 24 Stunden bei 20°C unter Inertgas (Argon) gerührt. Zum Aufarbeiten wurde die Reaktionsmischung über eine Fritte, beschickt mit Celite®, filtriert. Es wurde mit 100 ml $CH_2Cl_2$ nachgewaschen und das Filtrat am Rotationsverdampfer bei 45°C Badtemperatur eingedampft.

Das Rohprodukt wurde in 200 ml Diethylether gelöst, mit der gleichen Menge gesättigter $NaHSO_3$-Lösung versetzt und bei 20°C 24 Stunden gerührt. Anschließend wurde erneut über eine Fritte mit Celite® filtriert, mit 100 ml Diethylether nachgewaschen und die Phasen getrennt. Die wässrige Phase wurde einmal mit 100 ml Diethylether extrahiert, die vereinigten organischen Phasen 3 mal mit je 100 ml gesättiger Kochsalz lösung gewaschen, über Magnesiumsulfat getrocknet und am Rotationsverdampfer eingedampft.

B. Allgemeine Arbeitsvorschrift zur Herstellung von Nitroalkoholen der Formel (I) unter Verwendung von KF ohne Isopropanol.

In einen 50 ml-Kolben wurden 92 mMol eines Fluornitroalkans, 40 mMol einer Carbonylverbindung der Formel (III) und 3,4 mMol KF gegeben und unter Inertgas (Argon) 24 Stunden gerührt. Dann wurde das Reaktionsgemisch über eine mit Celite® beschickte Fritte filtriert, mit 100 ml $CH_2Cl_2$ nachgewaschen und am Rotationsverdampfer bei 45°C Badtemperatur eingedampft.

Das Rohprodukt wurde in 100 ml Diethylether gelöst, mit der gleichen Menge gesättigter $NaHSO_3$-Lösung versetzt und 24 Stunden bei 20°C gerührt. Anschließend wurde erneut über eine Fritte, die mit Celite® beschickt war filtriert und die Phasen getrennt. Die wässrige Phase wurde einmal mit 50 ml Diethylether extrahiert, die vereinigten organischen Phasen 3 mal mit je 50 ml gesättigter Kochsalzlösung gewaschen, über Magnesiumsulfat getrocknet und am Rotationsverdampfer eingedampft.

C. Allgemeine Arbeitsvorschrift zur Herstellung von Nitroalkoholen der Formel (I) unter Verwendung von $Al_2O_3$.

In einem 50 ml-Kolben unter Inertgas (Argon) wurden 24 mMol eines Fluornitroalkans gegeben, mit einem Eisbad gekühlt, mit der äquimolaren Menge einer Carbonylverbindung der Formel (III) versetzt und anschließend unter Rühren 5 g $Al_2O_3$ (neutral, Aktivitätsstufe I) zugegeben. Nach 5 Minuten wurde das Eisbad entfernt, 1 Stunde nachgerührt und dann 24 Stunden bei 20°C stehengelassen. Zum Aufarbeiten wurde das Reaktionsprodukt über eine mit Celite® beschickte Fritte filtriert, mit 50 ml $CH_2Cl_2$ nachgewaschen und das Filtrat am Rotationsverdampfer eingedampft.

Das Rohprodukt wurde in 100 ml Diethylether gelöst, mit der gleichen Menge gesättigter $NaHSO_3$-Lösung versetzt und 24 Stunden bei 20°C gerührt. Dann wurde über eine Fritte, beschickt mit Celite®, filtriert, die Phasen getrennt, die wässrige Phase 1 mal mit 50 ml Diethylether extrahiert, die vereinigten organischen Phasen 3 mal mit je 50 ml gesättigter Kochsalzlösung gewaschen, über Magnesiumsulfat getrocknet und am Rotationsverdampfer eingedampft.

D. Allgemeine Arbeitsvorschrift zur Herstellung von Nitroalkoholen unter Verwendung von Lithium-Alkyl-Verbindungen.

In einem 100 ml-Kolben mit seitlichem Ansatz, der mit einer Serumkappe verschlossen war und einem mit einem Dreiwegehahn versehenen Schliff aufwies, wurden 10 mMol eines Fluornitroalkans vorgelegt, mit 45 ml abs.THF, anschließend mit 15 ml abs.N,N-Dimethylpropylen-Harnstoff versetzt. Unter Rühren wurde mittels eines Kältebades (Methanol/flüssiger Stickstoff) auf -100 bis -110°C Innentemperatur abgekühlt und tropfenweise 22,4 mMol n-Butyl-Lithium (13,0 ml einer 1,59 molaren Lösung in n-Hexan) so zugegeben, daß die Innentemperatur -90°C nicht überstieg. Es wurden 15 -20 Minuten nachgerührt. Dann wurden bei -95°C 10 mMol einer Carbonylverbindung der Formel (III) zugegeben (bei Raumtemperatur kristalline Carbonylverbindungen in Form einer Lösung in 10 ml THF), wobei darauf geachtet wurde, daß die Temperatur -90°C nicht überstieg. Anschließend ließ man innerhalb von 45 Minuten auf -80 - -75°C aufwärmen, hielt für weitere 45 Minuten diese Temperatur und kühlte danach erneut auf -100 bis -110°C ab. Die so gekühlte Reaktionsmischung wurde tropfenweise mit einer Lösung von 3,5 ml Eisessig (61 mMol) und 3 ml THF so versetzt, daß die Innentemperatur nicht über -90°C anstieg. Dann ließ man innerhalb von einer Stunde auf -80 --70°C aufwarmen, entfernte das Kältbad und rührte für weitere 30 Minuten bei 20°C Außentemperatur, wobei die Innentemperatur auf +5 - +10°C anstieg.

Zum Aufarbeiten wurde das Reaktionsgemisch in ein Gemisch aus 75 ml Wasser und 75 ml Diethyl ether gegossen, die Phasen getrennt, die wässrige Phase einmal mit 75 ml Diethylether extrahiert, die vereinigten organischen Phasen 8 mal mit je 75 ml Wasser gewaschen, über Magnesiumsulfat getrocknet und am Rotationsverdampfer eingedampft.
Das so erhaltene Produkt wurde in 50 ml Diethylether gelöst, mit der gleichen Menge gesättigter $NaHSO_3$-Lösung versetzt und 24 Stunden bei 20°C gerührt. Anschließend wurde über eine Fritte, beschickt mit Celite®, filtiert, die Phasen getrennt, die wässrige Phase einmal mit 50 ml Diethylether extrahiert, die vereinigten organischen Phasen dreimal mit gesättigter Kochsalzlösung gewaschen, über Magnesiumsulfat getrocknet und am Rotationsverdampfer die flüchtigen Bestandteile abgezogen.

E. Allgemeine Arbeitsvorschrift zur katalytischen Hydrierung von fluorierten Nitroalkoholen der Formel (I) zu fluorierten Aminoalkoholen der Formel (IV).

In einem Erlenmeyer-Kolben wurden 5 g Raney-Nickel-Legierung in 50 ml Wasser aufgeschlämmt und portionsweise mit 8 g Natriumhydroxid versetzt. Danach wurde noch 1 Stunde bei 70 bis 80°C im Wasserbad erwärmt, anschließend mit 50 ml-Portionen Wasser neutral und anschließend mit Ethanol wasserfrei gewaschen. Das so erhaltene aktive Nickel wurde mit 50 ml Ethanol in einem 100 ml Autoklaven gespült, dazu wurde eine Lösung von 2 g des jeweiligen fluorierten Nitroalkohols der Formel (I) in 50 ml Ethanol gegeben, 25 bis 30 bar Wasserstoff aufgedrückt und 24 Stunden bei 50°C hydriert.

Anschließend wurde der Inhalt des Autoklaven über eine Fritte, beschickt mit Celite®, filtriert, mit 50 ml Ethanol nachgewaschen und am Rotationsverdampfer eingedampft.

Das so erhaltene Produkt wurde in 50 ml Diethylether gelöst, 3 mal mit je 50 ml 10%-iger wässriger Salzsäure extrahiert, die vereinigten wässrigen, salzsauren Phasen einmal mit 50 ml Diethylether gewaschen, anschließend unter Eiskühlung mit festem Natriumhydroxid versetzt bis die Lösung stark basisch war. Nun wurde 3 mal mit je 50 ml Diethylether extrahiert, die vereinigten organischen Phasen 2 mal mit je 50 ml gesättigter Kochsalzlösung gewaschen, über Magnesiumsulfat getrocknet und die flüchtigen Bestandteile am Rotationsverdampfer abgezogen.

Der so erhaltene Aminoalkohol wurde destilliert und als Oxalat charakterisiert.

II. Ausführungsbeispiele

Beispiele 1 bis 23:

Tabelle 3

| Beispiel Nr. | Reaktionsprodukt der Formel (I) X | R | $R_1$ | $R_2$ | allgem. Arbeitsvorschrift | Ausbeute in % roh (gereinigt) | Schmelz-(Fp) oder Siedepunkt (Kp) in °C | Diastereomeren-Verhältnis |
|---|---|---|---|---|---|---|---|---|
| 1 | F | H | H | Phenyl | A | (22) | $Kp_{0,6}$ : 80 - 85 | - |
| 2 | F | H | H | Phenyl | B | 10 | " | - |
| 3 | F | H | H | Phenyl | C | 9 | " | - |
| 4 | F | H | H | Phenyl | D | (39) | " | 2 : 1 |
| 5 | F | H | H | n-Propyl | A | 71 | $Kp_{12}$ : 55 - 65 | - |
| 6 | F | H | H | n-Propyl | B | 73 | " | - |
| 7 | F | H | H | n-Propyl | C | (50) | " | 3 : 1 |
| 8 | F | H | H | t-Butyl | D | (59) | $Kp_{12}$ : 70 - 80 | 2 : 1 |
| 9 | F | H | H | n-Hexyl | A | 65 | $Kp_{12}$ : 120 - 130 | - |
| 10 | F | H | H | n-Hexyl | B | (60) | " | 2 : 1 |

Tabelle 3 (Fortsetzung)

| Beispiel Nr. | Reaktionsprodukt der Formel (I) X | R | $R_1$ | $R_2$ | allgem. Arbeitsvorschrift | Ausbeute in % roh (gereinigt) | Schmelz-(Fp) oder Siedepunkt (Kp) in °C | Diastereomeren-Verhältnis |
|---|---|---|---|---|---|---|---|---|
| 11 | F | H | H | p-$NO_2$phenyl | D | 53 | Fp : 77,8 - 81,6 | 3 : 1 |
| 12 | F | H | H | 3,4-Methylendioxyphenyl | D | 40 | - | 2,5 : 1 |
| 13 | F | H | H | $-(CH_2)_2$-Phenyl | B | 83 | $Kp_{12}$ : 145 - 150 | 2,5 : 1 |
| 14 | F | H | H | $-CH-CH_3$<br>Phenyl | B | 46 | $Kp_{0,02}$: 80 | 2:1,5:3:1 |
| 15 | F | H | $-(CH_2)_5-$ | | D | 59 | $Kp_{12}$ : 95 | - |
| 16 | F | $CH_3$ | H | n-Propyl | A | (51) | $Kp_{12}$ : 80 - 90 | - |
| 17 | F | $CH_3$ | H | n-Propyl | B | 35 | " | 1,5 : 1 |
| 18 | F | $CH_3$ | H | n-Propyl | C | (25) | " | - |

Tabelle 3 (Fortsetzung)

| Beispiel Nr. | Reaktionsprodukt der Formel (I): X | R | $R_1$ | $R_2$ | allgem. Arbeitsvorschrift | Ausbeute in % roh (gereinigt) | Schmelz-(Fp) oder Siedepunkt (Kp) in °C | Diastereomeren-Verhältnis |
|---|---|---|---|---|---|---|---|---|
| 19 | F | $CH_3$ | H | n-Hexyl | A | (49) | $Kp_2$ : 80 - 90 | 2 : 1 |
| 20 | F | $CH_3$ | H | $-(CH_2)_2$-Phenyl | B | 40 | $Kp_{0,06}$ : 95 - 100 | 2 :1 |
| 21 | H | H | H | Phenyl | D | (12) | $Kp_{0,8}$ : 90 - 100 | 2 : 1 |
| 22 | H | H | H | t.-Butyl | D | 40 | $Kp_{12}$ : 95 - 105 | - |
| 23 | H | H | $-(CH_2)_5-$ | | D | 60 | $Kp_{12}$ : 115 - 120 | - |

Erläuterungen zur Tabelle 3

Das jeweils angegebene Reaktionsprodukt wurde durch Umsetzung eines Nitroalkans der Formel (II) mit X und R wie beim Reaktionsprodukt angegeben mit einer Carbonylverbindung der Formel (III) mit $R_2$ wie beim Reaktionsprodukt angegeben und $R_2$ = H erhalten. Ausnahme hiervon ist Beispiel 23, bei dem als Carbonylverbindung Cyclohexanon eingesetzt wurde.

Die Ausbeuten an Rohprodukt sind Auswaagen nach Entfernung des Lösungsmittels und beziehen sich in allen Fällen auf die Menge eingesetzter Carbonylverbindung. Die Ausbeuten an gereinigtem Produkt beziehen sich auf ein Produkt, das einmal destilliert wurde (Kugelrohrofen).

Alle Produkte zeigen die für Nitroalkohole charakteristische OH-Bande (breit, 3500 $cm^{-1}$) und $NO_2$-Bande (scharf, 1570 $cm^{-1}$), sowie intensive CF-Banden zwischen 1140 und 1260 $cm^{-1}$ im Infrarotspektrum (Film). Die $^1H$- und $^{13}C$-NMR-Spektren bestätigen die angegebenen Strukturen. Die Indexzahlen bei Kp geben den jeweiligen Druck (in Torr) an.

Das Diastereomeren-Verhältnis wurde aus dem $^{13}C$-NMR-Spektrum ermittelt.

Beispiele 24 bis 30:

Tabelle 4

| Beispiel Nr. | Reaktionsprodukt der Formel (IV) X | $R_3$ | $R_4$ | $R_5$ | Ausbeute in % | Schmelz-(Fp) oder Siedepunkt (Kp) in °C | Schmelzpunkt des jeweiligen Diaminomonooxalates in °C |
|---|---|---|---|---|---|---|---|
| 24 | F | H | H | n-Propyl | 52 | $Kp_{12}$ : 70 - 75 | 153,6 - 154,6 |
| 25 | F | H | H | n-Hexyl | 77 | $Kp_{12}$ : 115 | 158,5 - 159 |
| 26 | F | H | H | $-(CH_2)_2$-Phenyl | 79 | $Kp_{12}$ : 135 - 145<br>Fp : 40 - 50 | - |
| 27 | F | H | H | -CH-$CH_3$<br>Phenyl | 83 | $Kp_{0,01}$ : 70 - 75<br>Fp : 131 - 132 | 157 - 161 |
| 28 | F | $CH_3$ | H | n-Propyl | 5 | - | - |
| 29 | F | $CH_3$ | H | n-Hexyl | 22 | $Kp_2$ : 90 | - |
| 30 | F | $CH_3$ | H | $-(CH_2)_2$-Phenyl | 53,5 | $Kp_{0,8}$ : 90 - 100 | 158,4 - 160,6 |

Erläuterungen zur Tabelle 4:

Das jeweils angegebene Reaktionsprodukt wurde durch Hydrierung des jeweils entsprechenden fluorierten Nitroalkohols nach der allgemeinen Arbeitsvorschrift E erhalten.
Die Ausbeuten beziehen sich auf destillierte Produkte.
Das Diastereomen-Verhältnis der durch Destillation erhaltenen Aminoalkohole entspricht weitgehend

demjenigen der eingesetzten Nitroalkohole.

In den IR-Spektren (Film) der flüssigen Reaktionsprodukte erscheint jeweils eine breite, unstrukturierte Bande, im $OH/NH_2$-Bereich (3400 $cm^{-1}$) und die CF-Banden bei 1130 bis 1260 $cm^{-1}$, dazwischen liegen, außer bei Verbindungen, welche Arylgruppen enthalten, nur breite Banden geringerer Intensität.

Die $^{1}H$- und $^{13}C$-NMR-Spektren bestätigen die angegebenen Strukturen.

Die Indexzahlen bei Kp geben den jeweiligen Druck (in Torr) an.

In Beispiel 30 wurde der Schmelzpunkt des Monoaminomonooxalates bestimmt.

**Patentansprüche**

1. Fluorierte Nitroalkohole der Formel (I)

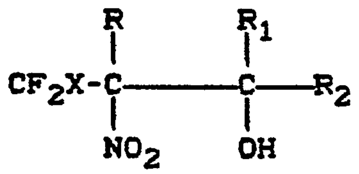

(I),

in der
X für Fluor oder Wasserstoff steht,
R für Wasserstoff oder $C_1$- bis $C_6$-Alkyl steht,
$R_1$ und $R_2$ unabhängig voneinander jeweils für Wasserstoff, gegebenenfalls substituiertes $C_1$- bis $C_6$-Alkyl, gegebenenfalls substituiertes $C_6$- bis $C_{10}$-Aryl oder gegebenenfalls substituiertes, 5 bis 7 Ringatome enthaltendes Heteroaryl stehen, wobei
$R_1$ und $R_2$ auch gemeinsam mit dem C-Atom, an das sie gebunden sind, für einen gegebenenfalls substituierten, gesättigten $C_5$- bis $C_7$-Ring stehen,
in den Fällen, in denen $R_1$ und $R_2$ identisch oder Teil eines unsubstituierten oder symmetrisch substituierten gesättigten $C_5$- bis $C_7$-Rings sind in der R- und S-Form und beliebigen Mischungen dieser Formen und in den Fällen, in denen $R_1$ und $R_2$ verschieden oder Teil eines unsymmetrisch substituierten gesättigten $C_5$- bis $C_7$-Rings sind, in der R,R-, R,S-, S,R- und S,S-Form und beliebigen Mischungen von zwei, drei und allen dieser Formen.

2. Fluorierte Nitroalkohole gemäß Anspruch 1, dadurch gekennzeichnet, daß in Formel (I)
X für Fluor,
R für Wasserstoff oder Methyl,
$R_1$ für Wasserstoff und
$R_2$ für $C_3$- bis $C_6$-Alkyl oder Phenyl steht oder
$R_1$ und $R_2$ gemeinsam mit dem C-Atom, an das sie gebunden sind, einen unsubstituierten gesättigten $C_6$-Ring bilden.

3. Verfahren zur Herstellung von fluorierten Nitroalkoholen der Formel (I)

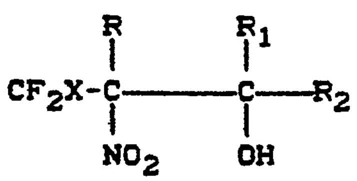

(I),

in der
X für Fluor steht,
R für Wasserstoff oder $C_1$- bis $C_6$-Alkyl steht,
$R_1$ und $R_2$ unabhängig voneinander jeweils für Wasserstoff, gegebenenfalls substituiertes $C_1$- bis $C_6$-Alkyl, gegebenenfalls substituiertes $C_6$- bis $C_{10}$-Aryl oder gegebenenfalls substituiertes, 5 bis 7 Ringatome enthaltendes Heteroaryl stehen, wobei
$R_1$ und $R_2$ auch gemeinsam mit dem C-Atom, an das sie gebunden sind, für einen gegebenenfalls substituierten, gesättigten $C_5$- bis $C_7$-Ring stehen,
dadurch gekennzeichnet, daß man Fluornitroalkane der Formel (II)

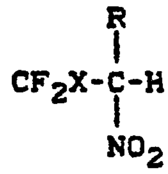 (II),

in der
X für Fluor steht
R die bei Formel (I) angegebene Bedeutung hat,
mit Carbonylverbindungen der Formel (III)

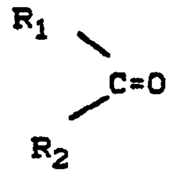 (III)

in der
$R_1$ und $R_2$ die bei Formel (I) angegebene Bedeutung haben,
in Gegenwart von Kaliumfluorid oder Aluminiumoxid umsetzt.

4. Verfahren nach Anspruch 3, dadurch gekennzeichnet, daß man Kaliumfluorid in Mengen von 0,05 bis 100 Mol-% oder Aluminiumoxid in Mengen von 0,1 bis 500 Mol-%, jeweils bezogen auf die Verbindung der Formel (II), einsetzt.

5. Verfahren zur Herstellung von fluorierten Nitroalkoholen der Formel (I)

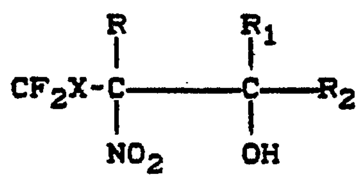 (I),

in der
X für Fluor oder Wasserstoff steht,
R für Wasserstoff steht,
$R_1$ und $R_2$ unabhängig voneinander jeweils für Wasserstoff, gegebenenfalls substituiertes $C_1$- bis $C_6$-Alkyl, gegebenenfalls substituiertes $C_6$- bis $C_{10}$-Aryl oder gegebenenfalls substituiertes, 5 bis 7 Ringatome enthaltendes Heteroaryl stehen, wobei
$R_1$ und $R_2$ auch gemeinsam mit dem C-Atom, an das sie gebunden sind, für einen gegebenenfalls substituierten, gesättigten $C_5$- bis $C_7$-Ring stehen,
dadurch gekennzeichnet, daß man Fluornitroalkane der Formel (II)

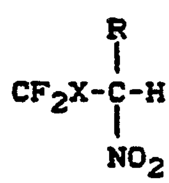 (II),

in der
X für Fluor oder Wasserstoff und
R für Wasserstoff steht
in Gegenwart eines aprotischen Lösungsmittels und gegebenenfalls eines Kosolvens mit einer Lithium-Alkyl-Verbindung bei Temperaturen im Bereich -80 bis -130°C umsetzt, dem Reaktionsgemisch anschließend bei -80 bis -130°C eine Carbonylverbindung der Formel (III)

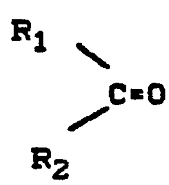 (III),

in der

$R_1$ und $R_2$ die bei Formel (I) angegebene Bedeutung haben,
zusetzt, anschließend auf -60 bis -80°C erwärmt, dann bei -80 bis -130°C mit Eisessig und einem aprotischen Lösungsmittel versetzt und abschließend auf eine Temperatur über -20°C erwärmt.

6. Fluorierte Aminoalkohole der Formel (IV)

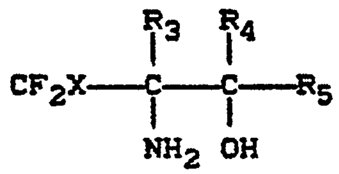

(IV)

in der
X für Fluor oder Wasserstoff steht,
$R_3$ für Wasserstoff oder $C_1$- bis $C_6$-Alkyl steht,
$R_4$ und $R_5$ unabhängig voneinander jeweils für Wasserstoff, gegebenenfalls substituiertes $C_1$- bis $C_6$-Alkyl, gegebenenfalls substituiertes $C_6$- bis $C_{10}$-Aryl oder gegebenenfalls substituiertes 5 bis 7 Ringatome enthaltendes Heteroaryl stehen,
wobei die Kombination X = F/$R_3$ =H/$R_4$ = H/$R_5$ = unsubstituiertes Phenyl ausgenommen ist.
und wobei $R_4$ und $R_5$ auch gemeinsam mit dem C-Atom, an das sie gebunden sind, für einen gegebenenfalls substituierten, gesättigten $C_5$- bis $C_7$-Ring stehen,
in den Fällen, in denen $R_4$ und $R_5$ identisch oder Teil eines unsubstituierten oder symmetrisch substituierten gesättigten $C_5$- bis $C_7$-Ringes sind, in der R- und S-Form und beliebigen Mischungen dieser Formen und in Fällen, in denen $R_4$ und $R_5$ verschieden oder Teil eines unsymmetrisch substituierten $C_5$- bis $C_7$-Ringes sind, in der R,R-, R,S-, S,R- und S,S-Form und beliebigen Mischungen von zwei, drei und allen vier dieser Formen.

7. Fluorierte Aminoalkohole gemäß Anspruch 6, dadurch gekennzeichnet, daß in Formel (IV)
$R_3$ für Wasserstoff oder Methyl,
$R_4$ für Wasserstoff und
$R_5$ für $C_3$- bis $C_6$-Alkyl oder, falls $R_3$ für Methyl steht, auch für Phenyl steht.

8. Verfahren zur Herstellung von fluorierten Aminoalkoholen der Formel (V)

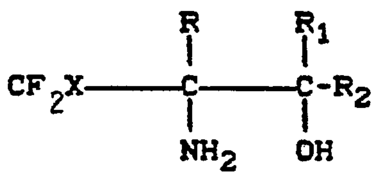

(V)

in der
X für Fluor oder Wasserstoff steht,
R für Wasserstoff oder $C_1$- bis $C_6$-Alkyl steht,
$R_1$ und $R_2$ unabhängig voneinander jeweils für Wasserstoff, gegebenenfalls substituiertes $C_1$- bis $C_6$-Alkyl, gegebenenfalls substituiertes $C_6$- bis $C_{10}$-Aryl oder gegebenenfalls substituiertes, 5 bis 7 Ringatome enthaltendes Heteroaryl stehen, wobei
$R_1$ und $R_2$ auch gemeinsam mit dem C-Atom, an das sie gebunden sind, für einen gegebenenfalls substituierten, gesättigten $C_5$- bis $C_7$-Ring stehen,
dadurch gekennzeichnet, daß man fluorierte Nitroalkohole der Formel (I)

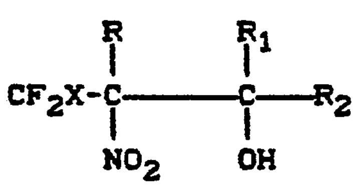

(I)

in der
X für Fluor oder Wasserstoff steht und
R, $R_1$ und $R_2$ die bei Formel (V) angegebene Bedeutung haben,
hydriert.

9. Verfahren nach Anspruch 8, dadurch gekennzeichnet, daß man die Hydrierung katalytisch mit Wasserstoff durchführt.

10. Verfahren nach Anspruch 8, dadurch gekennzeichnet, daß man die Hydrierung mit Metallhydriden durchführt.

**Revendications**

1. Nitroalcools fluorés de formule I

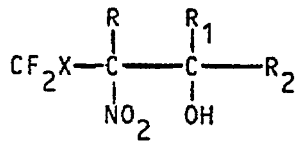

(I),

dans laquelle
X représente le fluor ou l'hydrogène,
R représente l'hydrogène ou un groupe alkyle en $C_1$–$C_6$,
$R_1$ et $R_2$ représentent chacun, indépendamment l'un de l'autre, l'hydrogène, un groupe alkyle en $C_1$–$C_6$ éventuellement substitué, un groupe aryle en $C_6$–$C_{10}$ éventuellement substitué, un groupe hétéroaryle contenant 5 à 7 atomes cycliques et éventuellement substitué,
$R_1$ et $R_2$ pouvant également former ensemble et avec l'atome de carbone auquel ils sont reliés un cycle saturé en $C_5$–$C_7$ éventuellement substitué,
sous les formes R et S et à l'état de mélanges quelconques de ces formes dans les cas où $R_1$ et $R_2$ sont identiques ou constituent une partie d'un cycle saturé en $C_5$–$C_7$ non substitué ou à substitution symétrique et sous les formes R, R; R, S; S, R et S, S et à l'état de mélanges quelconques de deux, trois et toutes ces formes dans les cas où $R_1$ et $R_2$ sont différents ou constituent une partie d'un cycle saturé en $C_5$–$C_7$ à substitution asymétrique.

2. Nitroalcools fluorés selon la revendication 1, caractérisés en ce que, dans la formule I
X représente le fluor,
R représente l'hydrogène ou un groupe méthyle,
$R_1$ représente l'hydrogène, et
$R_2$ représente un groupe alkyle en $C_3$–$C_6$ ou phényle, ou bien
$R_1$ et $R_2$ forment ensemble et avec l'atome de carbone auquel ils sont reliés un cycle saturé non substitué en $C_6$.

3. Procédé de préparation des nitroalcools fluorés de formule I

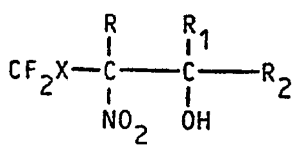

(I),

dans laquelle
X représente le fluor,
R représente l'hydrogène ou un groupe alkyle en $C_1$–$C_6$,
$R_1$ et $R_2$ représentent chacun, indépendamment l'un de l'autre, l'hydrogène, un groupe alkyle en $C_1$–$C_6$ éventuellement substitué, un groupe aryle en $C_6$–$C_{10}$ éventuellement substitué ou un groupe hétéroaryle contenant 5 à 7 atomes cycliques et éventuellement substitué,
$R_1$ et $R_2$ pouvant également former ensemble et avec l'atome de carbone auquel ils sont reliés un cycle saturé en $C_5$–$C_7$ éventuellement substitué,
caractérisé en ce que l'on fait réagir des fluoronitroalcanes de formule II

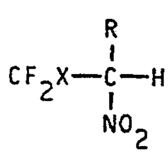

(II),

dans laquelle
X représente le fluor,
R a les significations indiquées en référence à la formule I, avec des composés carbonylés de formule III

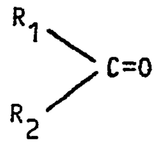

(III)

dans laquelle
$R_1$ et $R_2$ ont les significations indiquées en référence à la formule I, en présence de fluorure de potassium ou d'alumine.

4. Procédé selon la revendication 3, caractérisé en ce que l'on met en œuvre le fluorure de potassium en quantités de 0,05 à 100 moles % ou l'alumine en quantités de 0,1 à 500 moles %, dans les deux cas, par rapport au composé de formule II.

5. Procédé de préparation des nitroalcools fluorés de formule I

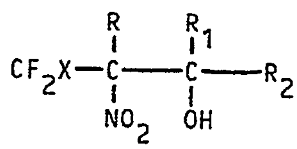

(I),

dans laquelle
X représente le fluor ou l'hydrogène,
R représente l'hydrogène,
$R_1$ et $R_2$ représentent chacun, indépendamment l'un de l'autre, l'hydrogène, un groupe alkyle en $C_1$–$C_6$ éventuellement substitué, un groupe aryle en $C_6$–$C_{10}$ éventuellement substitué ou un groupe hétéroaryle contenant 5 à 7 atomes cycliques et éventuellement substitué,
$R_1$ et $R_2$ pouvant également former ensemble et avec l'atome de carbone auquel ils sont reliés un cycle saturé en $C_5$–$C_7$ éventuellement substitué,
caractérisé en ce que l'on fait réagir des fluoronitroalcanes de formule II

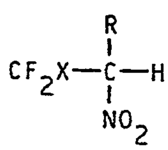

(II),

dans laquelle
X représente le fluor ou l'hydrogène, et
R représente l'hydrogène,
en présence d'un solvant aprotonique et éventuellement d'un cosolvant, avec un composé d'alkyl-lithium, à des températures dans l'intervalle de –80 à –130°C, on ajoute ensuite au mélange de réaction, entre –80 et –130°C, un composé carbonylé de formule III

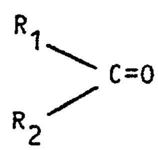

(III),

dans laquelle
$R_1$ et $R_2$ ont les significations indiquées en référence à la formule I, on réchauffe ensuite jusqu'à un niveau de –60 à –80°C puis on ajoute entre –80 et –130°C de l'acide acétique glacial et un solvant aprotonique et, pour terminer, on réchauffe à une température supérieure à –20°C.

6. Aminoalcools fluorés de formule IV

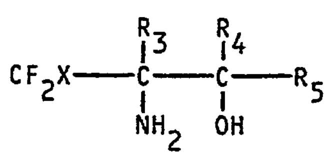

(IV)

dans laquelle
X représente le fluor ou l'hydrogène,
$R_3$ représente l'hydrogène ou un groupe alkyle en $C_1$–$C_6$,
$R_4$ et $R_5$ représentent chacun, indépendamment l'un de l'autre, l'hydrogène, un groupe alkyle en $C_1$–$C_6$ éventuellement substitué, un groupe aryle en $C_6$–$C_{10}$ éventuellement substitué ou un groupe hétéroaryle contenant 5 à 7 atomes cycliques et éventuellement substitué,
étant spécifié que la combinaison X = F/$R_3$= H/$R_4$ = H/$R_5$ = phényle non substitué, est exclue,
$R_4$ et $R_5$ pouvant également former ensemble et avec l'atome de carbone auquel ils sont reliés un cycle saturé en $C_5$–$C_7$ éventuellement substitué,
sous les formes R et S et à l'état de mélanges quelconques de ces formes dans les cas où $R_4$ et $R_5$ sont identiques ou constituent une partie d'un cycle saturé en $C_5$–$C_7$ non substitué ou à substitution symétrique, et sous les formes R,R; R,S; S,R et S,S et à l'état de mélanges quelconques de deux, trois ou ces quatre formes dans les cas où $R_4$ et $R_5$ sont différents ou constituent une partie d'un cycle en $C_5$–$C_7$ à substitution asymétrique.

7. Aminoalcools fluorés selon la revendication 6, caractérisés en ce que, dans la formule IV,
$R_3$ représente l'hydrogène ou un groupe méthyle,
$R_4$ représente l'hydrogène, et
$R_5$ représente un groupe alkyle en $C_3$–$C_6$, ou bien encore, lorsque $R_3$ représente un groupe méthyle, un groupe phényle.

8. Procédé de préparation des aminoalcools fluorés de formule V

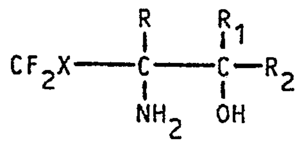 (V)

dans laquelle
X représente le fluor ou l'hydrogène,
R représente l'hydrogène ou un groupe alkyle en $C_1$–$C_6$,
$R_1$ et $R_2$ représentent chacun, indépendamment l'un de l'autre, l'hydrogène, un groupe alkyle en $C_1$–$C_6$ éventuellement substitué, un groupe aryle en $C_6$–$C_{10}$ éventuellement substitué ou un groupe hétéroaryle contenant 5 à 7 atomes cycliques et éventuellement substitué,
$R_1$ et $R_2$ pouvant également former ensemble et avec l'atome de carbone auquel ils sont reliés un cycle saturé en $C_5$–$C_7$ éventuellement substitué,
caractérisé en ce que l'on hydrogène des nitroalcools fluorés de formule I

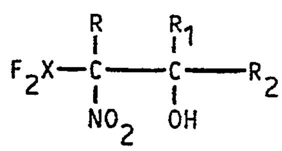 (I) .

dans laquelle
X représente le fluor ou l'hydrogène, et
R, $R_1$ et $R_2$ ont les significations indiquées en référence à la formule V.

9. Procédé selon la revendication 8, caractérisé en ce que l'hydrogénation est effectuée à l'aide d'hydrogène en présence d'un catalyseur.

10. Procédé selon la revendication 8, caractérisé en ce que l'hydrogénation est effectuée à l'aide d'hydrures métalliques.

**Claims**

1. Fluorinated nitroalcohols of the formula (I)

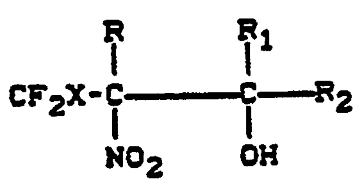 (I),

in which
X represents fluorine or hydrogen,
R represents hydrogen or $C_1$- to $C_6$-alkyl,
$R_1$ and $R_2$, independently of one another, in each case represent hydrogen, optionally substituted $C_1$– to $C_6$-alkyl, optionally substituted $C_6$- to $C_{10}$-aryl or optionally substituted heteroaryl containing 5 to 7 ring atoms,
where
$R_1$ and $R_2$, together with the C atom to which they are bound, alternatively represent an optionally substi-

tuted, saturated $C_5$- to $C_7$-ring, in the R and S form and any mixtures of these forms in the cases in which $R_1$ and $R_2$ are identical or part of an unsubstituted or symmetrically substituted saturated $C_5$- to $C_7$-ring, and in the R,R; R,S; S,R and S,S form and any mixtures of two, three and all of these forms in the cases in which $R_1$ and $R_2$ are different or part of an asymmetrically substituted saturated $C_5$- to $C_7$-ring.

2. Fulorinated nitroalcohols according to claim 1, characterized in that, in formula (I)
X represents fluorine,
R represents hydrogen or methyl,
$R_1$ represents hydrogen and
$R_2$ represents $C_3$- to $C_6$-alkyl or phenyl or
$R_1$ and $R_2$, together with the C atom to which they are bound, form an unsubstituted saturated $C_6$-ring.

3. Process for the preparation of fluorinated nitroalcohols of the formula (I)

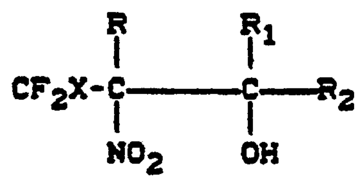
(I),

in which
X represents fluorine,
R represents hydrogen or $C_1$- to $C_6$-alkyl,
$R_1$ and $R_2$, independently of one another, in each case represent hydrogen, optionally substituted $C_1$- to $C_6$-alkyl, optionally substituted $C_6$- to $C_{10}$-aryl or optionally substituted heteroaryl containing 5 to 7 ring atoms,
where
$R_1$ and $R_2$, together with the C atom to which they are bound, alternatively represent an optionally substituted, saturated $C_5$- to $C_7$-ring, characterized in that fluoronitroalkanes of the formula (II)

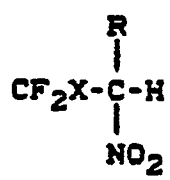
(II),

in which
X represents fluorine;
R has the meaning stated for formula (I),
are reacted with carbonyl compounds of the formula (III)

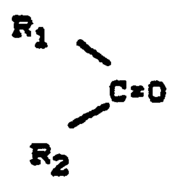
(III)

in which
$R_1$ and $R_2$ have the meaning stated for formula (I), in the presence of potassium fluoride or aluminium oxide.

4. Process according to claim 3, characterized in that potassium fluoride is employed in amounts from 0.05 to 100 mol % or aluminium oxide is employed in amounts from 0.1 to 500 mol %, in each case relative to the compound of the formula (II).

5. Process for the preparation of fluorinated nitroalcohols of the formula (I)

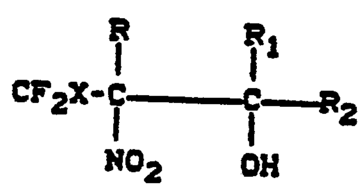
(I),

in which
X represents fluorine or hydrogen,

R represents hydrogen,
$R_1$ and $R_2$, independently of one another, in each case represent hydrogen, optionally substituted $C_1$- to $C_6$-alkyl, optionally substituted $C_6$- to $C_{10}$-aryl or optionally substituted heteroaryl containing 5 to 7 ring atoms,
where
$R_1$ and $R_2$, together with the C atom to which they are bound, alternatively represent an optionally substituted, saturated $C_5$- to $C_7$-ring,
characterized in that fluoronitroalkanes of the formula (II)

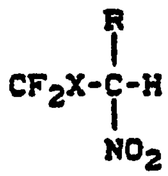
(II),

in which
X represents fluorine or hydrogen and
R represents hydrogen
are reacted with a lithium alkyl compound at temperatures in the range –80 to –130°C, in the presence of an aprotic solvent and, if appropriate, in the presence of a cosolvent, a carbonyl compound of the formula (III)

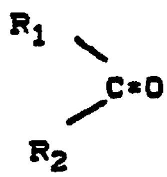
(III),

in which
$R_1$ and $R_2$ have the meaning stated for formula (I), is subsequently added to the reaction mixture at –80 to –130°C, the mixture is subsequently warmed to –60 to –80°C, glacial acetic acid and an aprotic solvent are then added at –80 to –130°C, and the mixture is subsequently warmed to a temperature above –20°C.

6. Fluorinated aminoalcohols of the formula (IV)

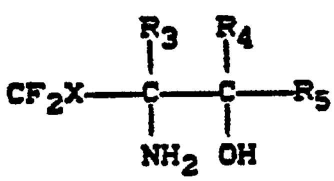
(IV)

in which
X represents fluorine or hydrogen,
$R_3$ represents hydrogen or $C_1$- to $C_6$-alkyl,
$R_4$ and $R_5$, independently of one another, in each case represent hydrogen, optionally substituted $C_1$- to $C_6$-alkyl, optionally substituted $C_6$- to $C_{10}$-aryl or optionally substituted heteroaryl containing 5 to 7 ring atoms,
where the combination X = F/$R_3$ = H/$R_4$ = H/$R_5$ = unsubstituted phenyl is excluded
and where $R_4$ and $R_5$, together with the C atom to which they are bound, alternatively represent an optionally substituted, saturated $C_5$- to $C_7$-ring,
in the R and S form and any mixtures of these forms in the cases in which $R_4$ and $R_5$ are identical or part of an unsubstituted or symmetrically substituted saturated $C_5$- to $C_7$-ring and in the R,R, R,S, S,R and S,S form and any mixtures of two, three or all four of these forms in the cases in which $R_4$ and $R_5$ are different or part of an asymmetrically substituted $C_5$- to $C_7$-ring.

7. Fluorinated aminoalcohols according to claim 6, characterized in that, in formula (IV)
$R_3$ represents hydrogen or methyl,
$R_4$ represents hydrogen and
$R_5$ represents $C_3$- to $C_6$-alkyl or, if $R_3$ represents methyl, alternatively represents phenyl.

8. Process for the preparation of fluorinated aminoalcohols of the formula (V)

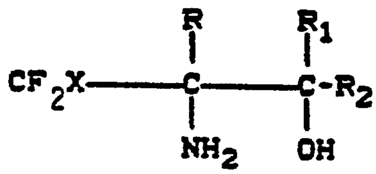

(V)

in which
X represents fluorine or hydrogen,
R represents hydrogen or $C_1$- to $C_6$-alkyl,
$R_1$ and $R_2$, independently of one another, in each case represent hydrogen, optionally substituted $C_1$- to $C_6$-alkyl, optionally substituted $C_6$- to $C_{10}$-aryl or optionally substituted heteroaryl containing 5 to 7 ring atoms,
where
$R_1$ and $R_2$, together with the C atom to which they are bound, alternatively represent an optionally substituted, saturated $C_5$- to $C_7$-ring, characterized in that fluorinated nitroalcohols of the formula (I)

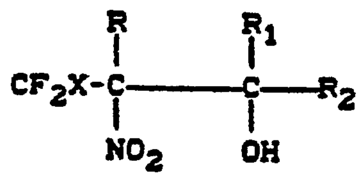

(I)

in which
X represents fluorine or hydrogen and
R, $R_1$ and $R_2$ have the meaning stated for formula (V),
are hydrogenated.

9. Process according to claim 8, characterized in that the hydrogenation is carried out catalytically using hydrogen.

10. Process according to claim 8, characterized in that the hydrogenation is carried out using metal hydrides.